# FASCICULE DE BREVET EUROPEEN

(11) **EP 2 705 866 B1**
(45) Date de publication et mention de la délivrance du brevet: **21.03.2018**
(21) Numéro de dépôt: 12183927.8
(22) Date de dépôt: 11.09.2012
(51) Int. Cl.: A61M 5/32

(54) **Dispositif pour réduire la douleur liée à l'introduction dans la peau d'une aiguille d'une seringue**
Vorrichtung zur Schmerzreduzierung bei der Einführung einer Spritzennadel in die Haut
Device for reducing pain related to the insertion of a syringe needle into the skin

(43) Date de publication de la demande: 12.03.2014
(73) Titulaire: Marti, Karim-Frédéric, 2525 Le Landeron (CH)
(72) Inventeur: Marti, Karim-Frédéric, 2525 Le Landeron (CH)
(74) Mandataire: Giraud, Eric

(56) Documents cités:
- WO-A1-2010/110823
- WO-A2-2008/081444
- WO-A2-2008/134580
- DE-A1-102010 046 560
- US-A1- 2010 022 965
- US-A1- 2010 174 237
- US-A1- 2010 211 010
- US-A1- 2012 065 487
- US-A1- 2012 130 340

## Description

La présente invention concerne un dispositif pour réduire la douleur liée à l'introduction dans la peau de l'aiguille d'une seringue.

Il est déjà connu de l'état de la technique plusieurs dispositifs susceptibles d'atténuer la douleur causée par la pénétration d'une aiguille d'une seringue dans la peau d'une personne pour l'injection d'une substance médicamenteuse. On peut citer à ce titre la demande de brevet internationale WO 98/18512 A1, qui décrit un tel dispositif permettant d'atténuer la douleur ressentie par une personne lors de la pénétration d'une aiguille d'une seringue à injection. Pour ce faire, ce dispositif présente principalement un anneau à une extrémité destiné à venir s'appuyer sur la peau d'un patient dans une zone où doit être effectuée l'injection. Cet anneau est porté par des tubes, qui sont reliés à une bague élastique pour définir une structure en forme de U. Cette bague élastique est montée à friction sur une paroi extérieure de la chambre logeant la seringue. Un cache pliable en plastique est également monté sur la structure en forme de U pour camoufler la vue de l'aiguille par le patient. L'anneau du dispositif est donc appuyé sur la peau du patient préalablement à l'injection de la substance médicamenteuse. La seringue peut ensuite coulisser dans la bague élastique suite à la pression pratiquée par l'utilisateur du dispositif de manière à faire pénétrer ladite aiguille dans la peau de la personne en passant à l'intérieur de l'anneau.

Ce type de dispositif décrit dans la demande de brevet internationale WO 98/18512 A1 comprend plusieurs éléments, qui ne sont pas faciles à monter les uns avec les autres de manière à d'une part cacher la vue de l'aiguille de seringue et d'autre part garantir un bon coulissement de la seringue jusqu'à la pénétration de l'aiguille dans la peau du patient. Cela constitue donc un inconvénient. De plus, ce dispositif n'est pas adapté pour bien s'appliquer sur une zone bien ciblée de la peau d'un patient, notamment il est mal adapté pour être utilisé dans la cavité buccale, ce qui constitue un autre inconvénient.

Dans le brevet CH 693 259, il est également décrit un dispositif pour réduire la douleur générée par l'aiguille d'une seringue à injection, lors de son introduction à travers la peau d'un patient. Pour ce faire, le dispositif comprend un manchon dans lequel peut être logé en partie le corps de la seringue avec l'aiguille. Une extrémité du manchon comprend une ouverture à travers laquelle peut passer l'aiguille de la seringue et une portion concave délimitée par un rebord annulaire pour venir s'appuyer sur une zone déterminée de la peau d'un patient pour une injection d'une substance médicamenteuse. Ce type de dispositif peut également être utilisé dans la cavité buccale d'un patient. Cependant le simple fait d'appliquer avec une certaine force le rebord de l'extrémité du manchon sur la peau d'un patient ne permet pas d'atténuer de manière suffisante la douleur générée par l'introduction de l'aiguille de seringue dans la peau du patient. Cela peut constituer un inconvénient.

Il est également connu des demandes de brevet US 2010/0211010 A1 et US 2010/0022965 A1 de prévoir un réservoir de substance anesthésiante sur le manchon recevant la seringue. La substance anesthésiante peut être giclée en direction de la peau d'une personne avant l'introduction de l'aiguille, mais cela ne permet pas d'insensibiliser précisément et localement l'endroit de la piqure, ce qui constitue un inconvénient.

Dans les demandes de brevet, DE 10 2010 046 560 A1, US 2010/0174237 A1 et WO 2008/081444 A2, il est décrit un moyen électrique disposé dans la partie terminale en contact de la peau pour effectuer une mesure par capteur ou pour insensibiliser la peau lors de l'introduction d'une aiguille, mais cela nécessite l'utilisation de moyens électriques supplémentaires, ce qui constitue un inconvénient.

Dans les demandes de brevet US 2012/0130340 A1 et WO 2008/134580 A2, il est défini une capsule flexible et une membrane flexible dans laquelle est disposé une substance médicamenteuse, qui est fournie lors de l'introduction de l'aiguille dans la peau. Cela ne permet pas de localement et facilement insensibiliser la peau de la personne, ce qui constitue un inconvénient.

L'invention a donc pour but de pallier aux inconvénients de l'état de la technique susmentionné en proposant un perfectionnement d'un dispositif pour réduire la douleur liée à l'introduction dans la peau de l'aiguille d'une seringue.

A cet effet, l'invention concerne un dispositif pour réduire la douleur liée à l'introduction dans le peau d'une aiguille d'une seringue, qui comprend les caractéristiques de la revendication indépendante 1.

Des formes d'exécution particulières du dispositif sont définies dans les revendications dépendantes 2 à 9.

Un avantage d'un tel dispositif de la présente invention réside dans le fait qu'il permet d'insensibiliser localement la peau d'un patient de manière mécanique et chimique, avant l'introduction d'une aiguille d'une seringue dans la peau. Cela améliore l'effet procuré par ces moyens de réduction de la douleur causée par une aiguille traversant la peau par rapport aux dispositifs de l'état de la technique. D'une part, la face avant de la partie terminale du manchon peut être pressée sur la peau d'une personne pour réduire la douleur à l'endroit de la pénétration de l'aiguille dans la peau. D'autre part, la substance active dans la cavité de la partie terminale est directement appliquée sur la peau du patient pour insensibiliser de manière chimique la peau à l'endroit de la piqûre.

Grâce à sa structure et son faible encombrement, le dispositif à seringue permet à un opérateur d'effectuer une injection à n'importe quel endroit du corps d'un patient, tout en ayant une main libre pour remplir des tâches annexes. Ainsi, il est possible avec le dispositif à seringue d'effectuer une injection au niveau des gencives du patient tout en lui assurant une sensation de douleur fortement réduite grâce au double effet d'insensibilisation selon l'invention.

A cet effet, l'invention concerne également un manchon pour un dispositif apte à réduire la douleur liée à l'introduction dans le peau d'une aiguille d'une seringue, selon la revendication indépendante 10.

L'invention sera mieux comprise à l'aide de la description suivante d'au moins une forme d'exécution du dispositif en référence aux dessins annexés, dans lesquels :
- la figure 1 représente une vue en perspective représentant le dispositif selon l'invention, où la seringue est partiellement insérée dans l'élément coiffant formant un manchon, et
- les figures 2a à 2c représentent trois vues en coupe partielle du dispositif selon l'invention représenté sur la figure 1 au niveau du manchon, dans une position initiale avant son utilisation, au moment de son utilisation et à l'instant où l'aiguille traverse la peau du patient.

Dans la description suivante, toutes les parties du dispositif, qui sont bien connues de l'homme du métier dans ce domaine technique, ne sont relatées que de manière simplifiée. La description suivante porte principalement sur le manchon ou élément coiffant du dispositif, qui comprend des moyens susceptibles de réduire la douleur liée à l'introduction d'une aiguille d'une seringue dans la peau d'une personne d'une manière mécanique et chimique.

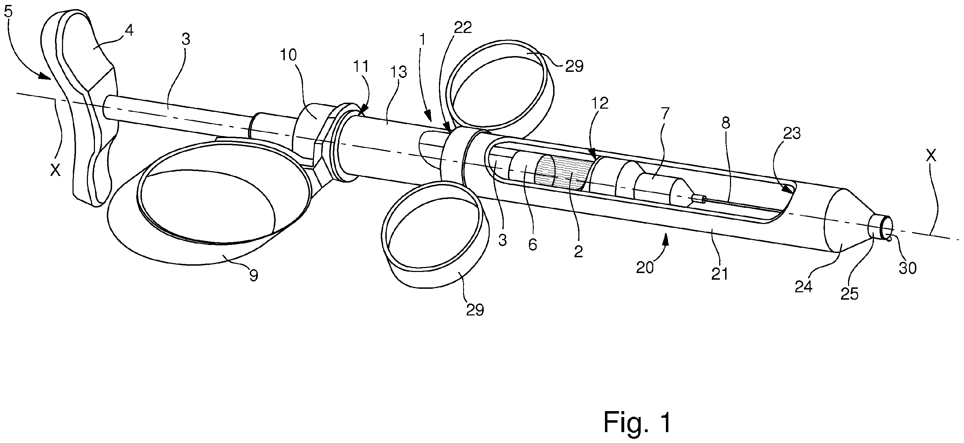

La figure 1 représente une vue tridimensionnelle d'un dispositif pour réduire la douleur liée à l'introduction dans le peau d'une aiguille 8 d'une seringue à injection 1. Cette forme d'exécution du dispositif reprend en grande partie des caractéristiques décrites dans la demande de brevet internationale WO 2004/043526, qui est incorporée ici par référence.

Sur cette figure 1 du dispositif, il est tout d'abord représenté une seringue 1 d'axe longitudinal X, qui comporte une chambre 2 destinée à contenir un liquide ou substance médicamenteuse à injecter à un patient. Une tige 3, qui porte un élément 4 présentant une surface d'appui 5 à son extrémité arrière, pénètre à l'intérieur de la chambre 2 de la seringue. La tige 3 porte à son extrémité avant un piston 6. Ce piston 6 est réalisé dans un matériau légèrement élastique et présente une section externe sensiblement supérieure à la section interne de la chambre 2, de telle manière qu'il assure l'étanchéité entre les deux parties de la chambre qu'il délimite. Ainsi, le piston 6 est susceptible de se déplacer en translation, dans la direction de l'axe X, pour transmettre la pression exercée par un utilisateur sur la surface d'appui 5 au liquide situé dans la chambre 2. Ainsi ce liquide ou substance médicamenteuse peut être expulsé en dehors de la seringue 1 lors d'une injection.

La seringue 1 comprend encore un embout 7, qui est vissé sur un filetage ou maintenu par friction à l'extrémité avant de la chambre 2. L'embout 7 porte une aiguille 8 et permet, de manière conventionnelle, d'adapter des aiguilles de différentes dimensions à la chambre 2. Un élément de préhension 9 de la seringue 1 a également été prévu au niveau de l'extrémité arrière de la chambre 2. Cet élément de préhension 9 peut présenter une forme d'anneau ou de bague comme représenté, qui est adaptée pour recevoir le pouce d'un utilisateur de la seringue.

La seringue 1 comprend également une fenêtre d'observation 12 ménagée dans une enveloppe 13 opaque dans laquelle la chambre 2 est disposée. Cette fenêtre d'observation 12 permet d'effectuer un contrôle visuel du niveau de liquide lors de l'injection.

La seringue 1 du dispositif est représentée sur la figure 1, alors qu'elle est partiellement insérée dans un espace intérieur délimité par un manchon ou élément coiffant 20. Le manchon 20 présente une première portion arrière principale 21 de forme générale cylindrique, dont la section interne est sensiblement supérieure à la section externe de la chambre 2 de la seringue 1, qui est constituée en partie par l'enveloppe 13. Le manchon 20 présente une ouverture circulaire 22, à son extrémité arrière, à travers laquelle la seringue 1 peut être en partie introduite avant une injection. La seringue 1 peut coulisser dans la première portion 21 dans une direction selon l'axe longitudinal X par l'intermédiaire notamment de son enveloppe 13 en contact avec la section interne de la première portion 21. La première portion principale 21 comporte en outre deux fenêtres 23 allongées et ménagées de manière à être diamétralement opposées. Ces fenêtres 23 sont prévues pour coopérer avec la fenêtre d'observation 12 de la seringue, lorsque cette dernière est en place dans l'espace intérieur du manchon 20.

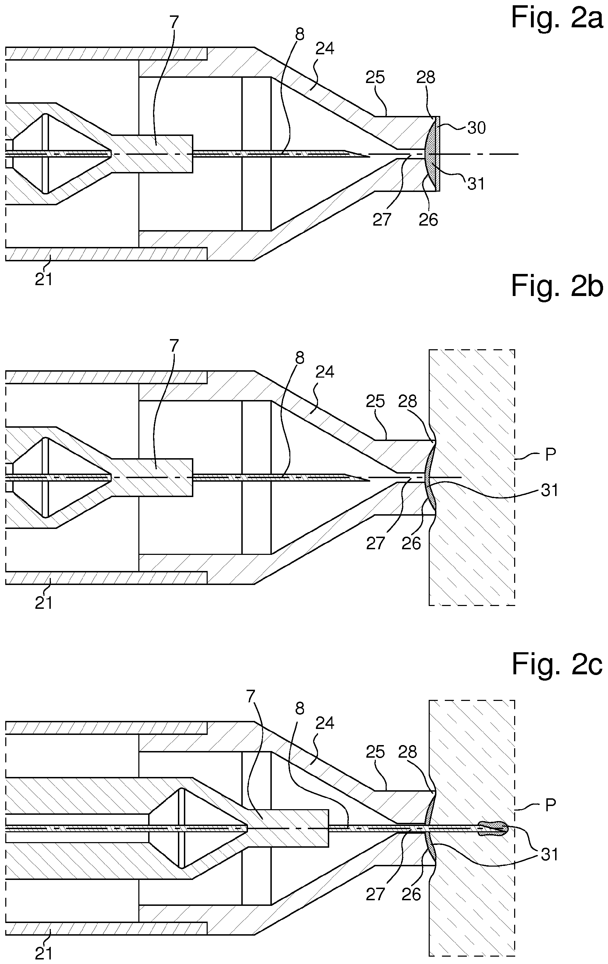

Le manchon 20 comporte encore une seconde portion 24, vers son extrémité avant en liaison de la première portion principale 21. Cette seconde portion est de forme tronconique et terminée par une partie terminale 25. La face avant de la partie terminale 25 peut présenter une faible superficie, comme il sera décrit plus en détail ci-après en référence aux figures 2a à 2c. Cette partie terminale est traversée dans la direction de l'axe X par une ouverture non représentée sur la figure 1, qui débute depuis la face avant et se termine dans l'espace intérieur de la première portion principale 21. Cette ouverture est dimensionnée pour laisser le passage à l'aiguille 8 de la seringue 1 pour une injection.

Selon la présente invention et comme décrit ci-après, la face avant de la partie terminale comprend encore une substance active, telle qu'un anesthésiant. Cette substance active ou médicale permet de rendre insensible l'endroit de la peau d'un patient à piquer par l'aiguille de la seringue en plus de la réduction de douleur par la pression mécanique de la face avant sur la peau du patient. Avant l'utilisation du dispositif avec ledit manchon 20, il peut être prévu de couvrir l'extrémité avant de la partie terminale 25 par un couvercle ou de préférence par un opercule 30, afin de préserver ladite substance des conditions environnementales.

Il est encore à noter que la chambre 2 sur son enveloppe 13, présente une courte portion 10 de section plus grande que sa section principale pour former un épaulement 11. Cette portion 10 est disposée à proximité de l'extrémité arrière de la chambre 2. Cet épaulement 11 a comme fonction de servir de butée contre l'extrémité arrière du manchon 20, pour limiter la course de la seringue 1 à l'intérieur du manchon 20. Par conséquent, l'épaulement 11 permet de limiter la longueur de l'aiguille 8 amenée à pénétrer dans la peau du patient.

Sur les figures 2a à 2c, il est représenté trois vues en coupe partielle longitudinale au niveau du manchon 20 du dispositif. Sur la première vue de la figure 2a, le dispositif avec le manchon 20 est représenté dans une position initiale avant utilisation. Sur la seconde vue de la figure 2b, le dispositif avec le manchon 20 est représenté au moment de son utilisation avec une partie de la face avant 26 de la partie terminale 25 du manchon 20 en contact de la peau P d'un patient. Sur la troisième vue de la figure 2c, le dispositif avec le manchon 20 est représenté à l'instant où l'aiguille 8 de la seringue traverse la peau P du patient.

Le manchon 20 comporte donc une première portion 21 à l'avant de laquelle est montée une seconde portion 24 de forme tronconique. Cette seconde portion 24 peut être fixée par collage ou soudure à la partie avant de la première portion 21 ou y être vissée ou également venir directement de matière avec cette première portion 21. Cette seconde portion 24 est terminée par une partie terminale 25 sous forme tubulaire. La première portion 21 et la seconde portion 24 avec la partie terminale 25 peuvent être réalisée dans un matériau métallique, voire un matériau plastique.

La partie terminale 25 de la seconde portion 24 comporte une face avant 26, dans laquelle débouche une ouverture 27. Cette ouverture 27 de forme tubulaire traverse entièrement la partie terminale 25 depuis la face avant jusqu'à l'espace intérieur de la première portion principale 21. Cette ouverture 27 est dimensionnée pour avoir un diamètre légèrement supérieur au diamètre de l'aiguille en section transversale de manière à laisser librement le passage à ladite aiguille 8 de la seringue.

La face avant 26 de cette partie terminale 25 est destinée à être mise en contact avec la peau P du patient peu de temps avant une injection. Cette face avant 26 comprend une partie en saillie agencée de manière continue sous la forme d'un rebord périphérique 28 autour de l'ouverture 27. Cette face avant 26 peut présenter selon un mode de réalisation représenté sur les figures 2a à 2c, une forme concave ou ovoïdale entre l'ouverture 27 et le rebord 28. Ledit rebord périphérique 28 peut être continu ou discontinu et avec une section transversale de forme semi-circulaire, carrée ou rectangulaire.

A titre d'exemple, le rebord périphérique 28 peut être dimensionné pour avoir une largeur l entre 0.1 et 0.3 mm, de préférence égale à 0.25 mm. Le diamètre de la partie terminale 25 peut être compris entre 4 et 8 mm, de préférence égal à 5 mm. Cependant d'autres dimensions peuvent être définies pour ces éléments, tout en permettant une utilisation du dispositif à seringue pour effectuer une injection au niveau du vestibule de la cavité buccale. Le dispositif à seringue doit donc être bien adapté pour de telles injections dans la région buccale, qui est particulièrement difficile d'accès.

La forme concave ou ovoïdale de la face avant 26 définit une cavité depuis le rebord périphérique 28 à l'ouverture 27. Selon la présente invention, il est prévu de disposer dans cette cavité une substance active 31, telle qu'un anesthésiant ou un réfrigérant. Cette substance active 31 est destinée à être appliquée sur la peau P d'un patient à l'endroit de la pénétration de l'aiguille 8 de la seringue. Cette substance active est appliquée sur une zone de la peau, où le rebord périphérique 28 est également mécaniquement pressé contre la peau du patient comme montré à la figure 2b. Ainsi un double effet d'insensibilisation de la peau intervient d'une part par la pression du rebord périphérique 28 de forme annulaire sur la peau et d'autre part par l'application de la substance sur la peau à l'endroit où l'aiguille 8 de la seringue est censée pénétrer dans la peau P du patient. Ceci permet donc de bien réduire la sensation de douleur liée à l'introduction dans la peau de ladite aiguille 8 par un effet chimique dû à ladite substance et par un effet mécanique grâce à la pression du rebord 28 contre la peau.

La substance active peut être sous forme de gel. Il peut s'agir d'un composé du type "Tropical Anesthetic Gel", par exemple le composé "Hurricaïne Tropical Anesthetic Gel", qui comprend par exemple 20% de Benzocaïne, de l'entreprise Beutlich LP Pharmaceuticals. Cependant d'autres types de substance active en tant qu'anesthésiant peuvent aussi convenir pour atténuer la douleur liée à l'introduction de l'aiguille d'une seringue dans la peau d'une personne. Il peut également être utilisé un composé chimique réfrigérant la peau du patient à son contact. Cette substance active 31 procure encore l'avantage d'être en partie entraînée par l'aiguille 8 de la seringue lors de son introduction dans la peau P comme montré par la figure 2c. Cela génère également un effet anesthésiant local directement sous la peau du patient.

Bien entendu, la cavité de la face avant 26 peut être aussi d'une autre forme, que celle présentée sur les figures 2a à 2c avec un fond plat ou légèrement incurvé et une ou plusieurs parois latérales se terminant par le rebord périphérique 28. Dans ces conditions, plus de substance médicale active 31 peut être logée dans ladite cavité. La face avant 26 peut aussi comporter une multitude de reliefs, qui sont soit concentriques, soit qui s'étendent dans la direction radiale depuis l'ouverture 27 jusqu'au rebord périphérique 28. Ceci permettrait de donner une bonne adhérence sur la face avant 26 à la substance active 31 disposée dans la cavité.

Avant son utilisation, la partie terminale 25 du manchon avec la substance active 31 disposée dans la cavité de la face avant 26, peut être recouverte par un couvercle traditionnel ou être fermée par un opercule 30 comme représenté sur la figure 2a. Cet opercule doit être retiré au moment de l'utilisation du dispositif. Ceci permet de préserver ladite substance active 31 par rapport aux conditions environnementales, dans lesquelles est conservé ledit dispositif à seringue. Par contre, il n'est pas forcément prévu de disposer une membrane friable sur l'ouverture 27, afin de ne pas émousser la pointe de l'aiguille 8 de la seringue à son passage.

A partir de la description qui vient d'être faite, plusieurs variantes du dispositif pour réduire la douleur liée à l'introduction dans le peau d'une aiguille d'une seringue peuvent être conçues par l'homme du métier sans sortir du cadre de l'invention définie par les revendications. Le dispositif à seringue peut être utilisé pour effectuer des prélèvements sanguins au lieu d'effectuer des injections tout en bénéficiant du double effet mécanique et chimique d'insensibilisation de la peau. Le dispositif à seringue peut être prévu pour faciliter une injection dans la cavité buccale d'un patient. Il peut être prévu de pratiquer des rainures rectilignes, courbées ou circulaires autour de la seconde ouverture de la face avant pour disposer la substance active. Cette substance active peut se présenter également sous forme de liquide ou de poudre.

## Revendications

1. Dispositif pour réduire la douleur liée à l'introduction dans la peau d'une aiguille (8) d'une seringue, le dispositif comprenant encore un manchon (20), qui comporte une première portion arrière (21) pour recevoir au moins partiellement la seringue (1) avec l'aiguille (8), et une seconde portion avant (24) munie d'une partie terminale (25) traversée par une ouverture (27) agencée pour le passage de l'aiguille (8), une face avant (26) de la partie terminale (25) étant destinée à être appliquée avec pression contre la peau d'une personne avant l'introduction de l'aiguille (8) dans la peau,
**caractérisé en ce que** la face avant (26) de la partie terminale (25) comprend une cavité entre l'ouverture (27) et un rebord périphérique (28) de la partie terminale (25), une substance active ou médicale (31) d'insensibilisation de la peau étant disposée dans la cavité de la face avant (26) de la partie terminale (25) de telle manière à être directement appliquée sur la peau du patient à l'endroit de la piqûre et être entraînée par l'aiguille (8) de la seringue lors de son introduction dans la peau du patient.

2. Dispositif selon la revendication 1, **caractérisé en ce que** la substance active (31) est une substance anesthésiante.

3. Dispositif selon la revendication 2, **caractérisé en ce que** la substance anesthésiante (31) est sous forme de gel.

4. Dispositif selon la revendication 1, **caractérisé en ce que** la substance active (31) est une substance réfrigérante.

5. Dispositif selon la revendication 1, **caractérisé en ce qu'**un opercule (30) est fixé sur le rebord périphérique (28) pour enfermer la substance active (31) dans la cavité de la face avant (26) de la partie terminale.

6. Dispositif selon la revendication 1, **caractérisé en ce que** la substance active (31) est disposée dans toute la cavité depuis l'ouverture (27) au rebord périphérique (28).

7. Dispositif selon la revendication 1, **caractérisé en ce que** la cavité a une forme concave ou ovoïdale entre l'ouverture (27) et le rebord périphérique (28) de forme annulaire.

8. Dispositif selon la revendication 1, **caractérisé en ce que** la face avant (26) de la partie terminale (25) comprend des rainures rectilignes, courbées ou circulaires, dans lesquelles la substance active (31) est disposée.

## Patentansprüche

1. Vorrichtung zum Verringern des Schmerzes, der mit der Einführung einer Nadel (8) einer Spritze in die Haut verbunden ist, wobei die Vorrichtung ferner eine Hülse (20) enthält, die einen ersten, hinteren Abschnitt (21) für die zumindest teilweise Aufnahme der Spritze (1) mit der Nadel (8) und einen zweiten, vorderen Abschnitt (24) aufweist, der mit einem Endteil (25) versehen ist, durch den eine Öffnung (27) verläuft, die für den Durchgang der Nadel (8) ausgelegt ist, wobei eine vordere Fläche (26) des Endteils (25) dazu bestimmt ist, mit Druck auf die Haut einer Person aufgesetzt zu werden, bevor die Nadel (8) in die Haut eingeführt wird,
**dadurch gekennzeichnet, dass** die vordere Fläche (26) des Endteils (25) zwischen der Öffnung (27) und einem Umfangsrand (28) des Endteils (25) einen Hohlraum aufweist, wobei eine aktive oder medizinische Substanz (31) für die Betäubung der Haut in dem Hohlraum der vorderen Fläche (26) des Endteils (25) angeordnet ist, derart, dass sie direkt auf die Haut des Patienten an der Stelle des Einstichs aufgebracht wird und durch die Nadel (8) der Spritze bei deren Einführung in die Haut des Patienten mitgenommen wird.

2. Vorrichtung nach Anspruch 1, **dadurch gekennzeichnet, dass** die aktive Substanz (31) eine betäubende Substanz ist.

3. Vorrichtung nach Anspruch 2, **dadurch gekennzeichnet, dass** die betäubende Substanz (31) in Form eines Gels vorliegt.

4. Vorrichtung nach Anspruch 1, **dadurch gekennzeichnet, dass** die aktive Substanz (31) eine kühlende Substanz ist.

5. Vorrichtung nach Anspruch 1, **dadurch gekennzeichnet, dass** an dem Umfangsrand (28) eine Abdeckung (30) befestigt ist, um die aktive Substanz (31) in den Hohlraum der vorderen Fläche (26) des Endteils einzuschließen.

6. Vorrichtung nach Anspruch 1, **dadurch gekennzeichnet, dass** die aktive Substanz (31) in dem gesamten Hohlraum von der Öffnung (27) bis zu dem Umfangsrand (28) angeordnet ist.

7. Vorrichtung nach Anspruch 1, **dadurch gekennzeichnet, dass** der Hohlraum zwischen der Öffnung (27) und dem ringförmigen Umfangsrand (28) konkav oder eiförmig ist.

8. Vorrichtung nach Anspruch 1, **dadurch gekennzeichnet, dass** die vordere Fläche (26) des Endteils (25) geradlinige, gekrümmte oder kreisförmige Rillen besitzt, in denen die aktive Substanz (31) angeordnet ist.

## Claims

1. Device for reducing the pain associated with the insertion of a needle (8) of a syringe into the skin, wherein the device comprises a sleeve (20), which has a first rear portion (21) to at least partially receive the syringe (1) with the needle (8) and a second front portion (24) fitted with an end part (25), through which an opening (27) passes for passage of the needle (8), wherein a front face (26) of the end part (25) is intended to be applied with pressure against the skin of a person before insertion of the needle (8) into the skin,
**characterized in that** the front face (26) of the end part (25) comprises one cavity between the opening (27) and a peripheral edge (28) of the end part (25), an active or medicinal substance (31) for desensitizing the skin being arranged in the cavity of the front face (26) of the end part (25) to be applied directly onto the skin of the patient at the penetration site, and to be entrained by the needle (8) of the syringe during insertion into the skin of the patient.

2. Device according to claim 1, **characterized in that** the active substance (31) is an anesthetic substance.

3. Device according to claim 2, **characterized in that** the anesthetic substance (31) is in the form of a gel.

4. Device according to claim 1, **characterized in that** the active substance (31) is a cooling substance.

5. Device according to claim 1, **characterized in that** a protective cap (30) is fixed onto the peripheral edge (28) to enclose the active substance (31) in the cavity of the front face (26) of the end part.

6. Device according to claim 1, **characterized in that** the active substance (31) is disposed in the whole cavity from the opening (27) to the peripheral edge (28).

7. Device according to claim 1, **characterized in that** the cavity is concave or ovoid in shape between the opening (27) and the ring-shaped peripheral edge (28).

8. Device according to claim 1, **characterized in that** the front face (26) of the end part (25) comprises rectilinear, curved or circular grooves, in which the active substance (31) is disposed.
